# EUROPÄISCHE PATENTSCHRIFT

(11) **EP 2 754 421 B1**
(45) Veröffentlichungstag und Bekanntmachung des Hinweises auf die Patenterteilung: **12.08.2015**
(21) Anmeldenummer: 14164425.2
(22) Anmeldetag: 02.03.2009
(51) Int. Cl.: A61F 2/38

(54) **Medizinisches Implantat und Kniegelenkendoprothese**
Medical implant and knee joint endoprosthesis
Implant médical et prothèse d'articulation du genou

(30) Priorität: 07.03.2008 DE 102008014837
(43) Veröffentlichungstag der Anmeldung: 16.07.2014
(62) Teilanmeldung aus: 09716618.5
(73) Patentinhaber: AESCULAP AG, 78532 Tuttlingen (DE)
(72) Erfinder: Moussa, Said, 52000 Chamarandes-Choignes (FR)
(74) Vertreter: Hoeger, Stellrecht & Partner Patentanwälte mbB

(56) Entgegenhaltungen:
- EP-A- 0 376 658
- WO-A1-2007/114841
- DE-U1-202007 008 538
- US-A- 5 061 271
- US-A1- 2003 055 508
- US-B1- 6 699 290

## Beschreibung

Die Erfindung betrifft ein medizinisches Implantat mit einem ersten Implantatteil und einem zweiten Implantatteil, zu deren Verbindung eine Verbindungseinrichtung vorgesehen ist, wobei zumindest das erste Implantatteil eine Verbindungselementaufnahme aufweist, in der mindestens ein Verbindungselement der Verbindungseinrichtung anordenbar ist, wobei eine Sicherungseinrichtung zur Sicherung des mindestens einen Verbindungselementes in der Verbindungselementaufnahme vorgesehen ist, wobei die Sicherungseinrichtung mindestens ein Sicherungsglied zur Sicherung des mindestens einen Verbindungselementes in der Verbindungselementaufnahme umfasst.

Außerdem betrifft die Erfindung eine Kniegelenkendoprothese, umfassend mindestens ein Implantat mit einem ersten Implantatteil und einem zweiten Implantatteil, zu deren Verbindung eine Verbindungseinrichtung vorgesehen ist, wobei zumindest das erste Implantatteil eine Verbindungselementaufnahme aufweist, in der mindestens ein Verbindungselement der Verbindungseinrichtung anordenbar ist.

Zur Verbindung des ersten Implantatteils mit dem zweiten Implantatteil weist die Verbindungseinrichtung mindestens ein Verbindungselement auf, das in der Verbindungselementaufnahme des ersten Implantatteils anordenbar ist. Dieses erste Verbindungselement der Verbindungseinrichtung kann zum Beispiel mit einem zweiten Verbindungselement der Verbindungseinrichtung zusammenwirken, welches beispielsweise am zweiten Implantatteil anordenbar oder angeordnet ist.

Ein Implantat ist in der DE 20 2007 008 538 U1 beschrieben und umfasst ein ein Tibiaplateau definierendes und teilweise eine Verbindungseinrichtung umfassendes erstes Implantatteil sowie ein zweites Implantatteil in Form eines Tibiaschaftes. Die Verbindungseinrichtung weist eine nutförmige Verbindungselementaufnahme auf, in die ein erstes Verbindungselement in Form einer Mutter einführbar und anschließend mit einem am Tibiaschaft angeordneten zweiten Verbindungselement in Form einer Gewindespindel verschraubt werden kann, um die beiden Implantatteile miteinander zu verbinden.

Eine Kniegelenkendoprothese ist in der US 2003/0055508 A1 beschrieben. Die Prothese umfasst einen Tibiaschaft, ein Tibiaplateau und einen diese verbindenden Adapter. Der Tibiaschaft kann mit dem Adapter verbunden werden, indem ein Verbindungselement am Schaft mit einem Verbindungselement am Adapter zusammenwirkt. Der Schaft wird in eine Aufnahme des Adapters eingeführt. Quer zur Einführrichtung wird ein Verbindungselement in den Adapter eingeführt, das mit dem am Schaft angeordneten Verbindungselement koppelt.

Eine weitere Kniegelenkendoprothese ist in der WO 2007/114841 A1 beschrieben.

Aufgabe der vorliegenden Erfindung ist es, ein gattungsgemäßes Implantat sowie eine gattungsgemäße Kniegelenkendoprothese bereitzustellen, das beziehungsweise die eine einfachere Handhabbarkeit aufweist.

Diese Aufgabe wird bei einem Implantat der eingangs genannten Art erfindungsgemäß durch die Merkmale des kennzeichnenden Teils von Anspruch 1 gelöst.

Mittels der Sicherungseinrichtung kann das mindestens eine Verbindungselement in der Verbindungselementaufnahme gesichert werden. Dadurch kann vermieden werden, dass sich das mindestens eine Verbindungselement unbeabsichtigterweise aus der Verbindungselementaufnahme löst, zum Beispiel aus ihm wegbewegt. Dies wirkt sich verbessernd auf die Handhabbarkeit des Implantates aus. So kann zum Beispiel vor der Verbindung des ersten Implantatteiles mit dem zweiten Implantatteil das mindestens eine Verbindungselement in die Verbindungselementaufnahme eingeführt und mit der Sicherungseinrichtung, welche dabei beispielsweise eine Sicherungsstellung einnehmen kann, darin gesichert werden. Dadurch kann ein Benutzer sein Augenmerk auf das Verbinden des ersten Implantatteiles mit dem zweiten Implantatteil richten, ohne zusätzlich auf die Sicherung des mindestens einen Verbindungselementes in der Verbindungselementaufnahme achten zu müssen. Bei bestehender Verbindung des ersten Implantatteiles mit dem zweiten Implantatteil kann sich die Sicherung des mindestens einen Verbindungselementes in der Verbindungselementaufnahme fördernd auf die Zuverlässigkeit der Verbindung beider Implantatteile auswirken. Die Sicherung kann beispielsweise durch eine Halterung des mindestens einen Verbindungselementes in der Verbindungselementaufnahme sichergestellt werden, wobei unter "Halterung" vorliegend sowohl eine festgelegte Halterung als auch eine bewegliche Halterung verstanden werden kann.

Die Sicherungseinrichtung umfasst mindestens ein Sicherungsglied zur Sicherung des mindestens einen Verbindungselementes in der Verbindungselementaufnahme in einer Sicherungsstellung der Sicherungseinrichtung. Das mindestens eine Sicherungsglied ist zur Erzielung einer kompakten Bauform des Implantates günstigerweise am ersten Implantatteil und/oder an der Verbindungseinrichtung angeordnet, wobei es insbesondre an dem mindestens einen Verbindungselement angeordnet sein kann.

Von Vorteil ist es, dass die Verbindungselementaufnahme mindestens eine Einführöffnung für das mindestens eine Verbindungselement aufweist, die mit dem mindestens einen Sicherungsglied zumindest teilweise verschlossen ist. Dies gibt auf technisch einfache Weise die Möglichkeit, das mindestens eine Verbindungselement in der Verbindungselementaufnahme zu sichern. Mittels des mindestens einen Sicherungsgliedes ist die Einführöffnung zumindest teilweise so verschließbar, dass deren verbleibende Querschnittsfläche kleiner ist als die Querschnittsfläche des mindestens einen Verbindungselementes, so dass dieses nicht aus der Verbindungselementaufnahme wegbewegt werden kann.

Günstig ist es, wenn die Sicherungseinrichtung am ersten Implantatteil angeordnet ist und/oder davon umfasst wird, denn dadurch lässt sich eine kompakte Bauform des Implantates erreichen.

Von Vorteil ist es, wenn die Sicherungseinrichtung an der Verbindungseinrichtung angeordnet ist und/oder davon umfasst wird, wobei insbesondere vorgesehen sein kann, dass die Sicherungseinrichtung an dem mindestens einen Verbindungselement angeordnet ist und/oder davon umfasst wird. Auch auf diese Weise lässt sich eine kompakte Bauform des Implantates erzielen.

Vorzugsweise ist dem mindestens einen Verbindungselement ein Anlageglied zugeordnet, an welches es zum Einführen in die Verbindungselementaufnahme anlegbar ist. Dies erleichtert einem Benutzer das Einführen des mindestens einen Verbindungselementes in die Verbindungselementaufnahme und verbessert so die Handhabbarkeit des Implantates. Das Anlageglied kann zum Beispiel eine Anlagefläche für das mindestens eine Verbindungselement ausbilden, an welche dieses zum Einführen in die Verbindungselementaufnahme anlegbar ist. Es kann auch vorgesehen sein, dass das mindestens eine Verbindungselement am Anlageglied anliegend mit diesem zusammen in die Verbindungselementaufnahme eingeführt werden kann.

Um eine kompakte Bauform des Implantates zu erzielen, umfasst vorzugsweise die Sicherungseinrichtung das mindestens eine Anlageglied.

Besonders günstig ist es, wenn die mindestens eine Einführöffnung mittels des mindestens einen Sicherungsgliedes vollständig oder im Wesentlichen vollständig verschließbar ist. Dies erlaubt es, das mindestens eine Verbindungselement noch zuverlässiger in der Verbindungselementaufnahme zu sichern.

Vorzugsweise ist die mindestens eine Einführöffnung in einem Wandabschnitt des ersten Implantatteiles gebildet, und das mindestens eine Sicherungsglied fluchtet, zum Beispiel in einer Sicherungsstellung der Sicherungseinrichtung, zumindest abschnittsweise mit dem Wandabschnitt oder fluchtet im Wesentlichen mit dem Wandabschnitt. Dies erlaubt es, einen gleichmäßigen Übergang von dem Wandabschnitt zu dem mindestens einen Sicherungsglied zu erzielen. Diese können dadurch eine gemeinsame Fläche bilden, wobei sich das mindestens eine Sicherungsglied bevorzugt fügespaltfrei oder im Wesentlichen fügespaltfrei in die Einführöffnung einfügt. Dadurch lässt sich nicht nur das mindestens eine Verbindungselement zuverlässig in der Verbindungselementaufnahme sichern, sondern es ist auch die Möglichkeit gegeben, dass außerhalb der Verbindungselementaufnahme liegendes Material nicht oder nur mit geringer Wahrscheinlichkeit in die Verbindungselementaufnahme eindringen kann. Bei einem derartigen Material handelt es sich zum Beispiel um Knochenzement, welcher zur Festlegung des Implantates im Körper eines Patienten eingesetzt wird. Durch einen fluchtenden oder im Wesentlichen fluchtenden Übergang zwischen dem Wandabschnitt und dem mindestens einen Sicherungsglied lassen sich überdies Hinterschneidungen im Bereich der mindestens einen Einführöffnung vermeiden, in denen sich unerwünschterweise Knochenzement ansammeln kann.

Von Vorteil ist es, wenn die Verbindungselementaufnahme zwei Einführöffnungen zum Einführen des mindestens einen Verbindungselementes umfasst und wenn die Sicherungseinrichtung zwei Sicherungsglieder aufweist, mit denen jeweils eine Einführöffnung zumindest abschnittsweise und noch vorteilhafter vollständig oder im Wesentlichen vollständig verschließbar ist. Das Vorhandensein von zwei Einführöffnungen erleichtert einem Benutzer das Einführen des mindestens einen Verbindungselementes in die Verbindungselementaufnahme. Je nach Zugänglichkeit des Implantates während einer Operation kann sich die eine oder die andere Einführöffnung als besser geeignet zum Einführen des mindestens einen Verbindungselementes erweisen. Um einen möglichst großen Raumwinkel abzudecken, ist es günstig, wenn die zwei Einführöffnungen an einander gegenüberliegenden Seiten der Verbindungselementaufnahme gebildet sind.

Vorzugsweise sind die zwei Sicherungsglieder miteinander verbunden. Dies erlaubt eine technisch einfache Konstruktion der Sicherungseinrichtung, da keine separaten Sicherungsglieder vorgehalten werden müssen, die eventuell verlorengehen könnten. Stattdessen sind die zwei Sicherungsglieder miteinander verbunden, und es ist insbesondere möglich, dass die Sicherungseinrichtung einteilig ausgebildet ist. Die Verbindung der zwei Sicherungsglieder miteinander kann bevorzugt über das vorstehend beschriebene Anlageglied der Sicherungseinrichtung erfolgen, an welches das mindestens eine Verbindungselement zum Einführen in die Verbindungselementaufnahme anlegbar ist.

Eine einfache Konstruktion der Sicherungseinrichtung kann dadurch erzielt werden, dass die Sicherungseinrichtung eine Symmetrie aufweist, aufgrund derer zumindest die zwei Sicherungsglieder relativ zueinander symmetrisch ausgebildet sind, wobei es noch günstiger ist, wenn die gesamte Sicherungseinrichtung symmetrisch ausgebildet ist. Bei dieser Ausführungsform ist es darüber hinaus von Vorteil, wenn die Verbindungselementaufnahme ebenfalls symmetrisch ausgebildet ist. Damit kann erreicht werden, dass sich jede Einführöffnung mit jeden der beiden Sicherungsglieder zumindest teilweise verschließen lässt. Dadurch wird die Handhabbarkeit des Implantates verbessert.

Vorzugsweise bildet die Sicherungseinrichtung eine von einer oder mehreren Wandungen zumindest teilweise begrenzte Sicherungsaufnahme für das mindestens eine Verbindungselement aus. Über die Verbindungselementaufnahme hinaus kann das mindestens eine Verbindungselement in der Sicherungsaufnahme angeordnet und darin gesichert sein, zum Beispiel um es in die Verbindungselementaufnahme einzuführen. An die eine oder die mehreren Wandungen der Sicherungseinrichtung kann das mindestens eine Verbindungselement hierzu beispielsweise angelegt werden.

Besonders günstig ist es, wenn mindestens eine der einen oder mehreren Wandungen der Sicherungseinrichtung die Verbindungselementaufnahme zumindest abschnittsweise begrenzt. Auf diese Weise hat die eine oder die mehreren Wandungen eine doppelte Funktion, denn sie begrenzt zumindest teilweise sowohl die Sicherungsaufnahme als auch die Verbindungselementaufnahme. Bei einer derartigen Wandung handelt es sich besonders bevorzugt um das vorstehend beschriebene mindestens eine Sicherungsglied der Sicherungseinrichtung.

Es kann vorgesehen sein, dass das mindestens eine Verbindungselement in der Sicherungsaufnahme beweglich angeordnet und insbesondere beweglich gelagert ist. Dies erlaubt es, das mindestens eine Verbindungselement beweglich am ersten Implantatteil zu sichern, so dass dadurch die Möglichkeit gegeben ist, dass das mindestens eine Verbindungselement relativ zum zweiten Implantatteil eine Mehrzahl von Positionen einnehmen kann. Dies kann beim Einsetzen des Implantates in den Körper eines Patienten von Vorteil sein, um die Ausrichtung der beiden Implantatteile relativ zueinander auf die Bedürfnisse des jeweiligen Patienten einzustellen.

Auf besonders einfache Weise ist das Implantat handhabbar, wenn die Sicherungseinrichtung ausgehend von einer Montagestellung, in der das mindestens eine Verbindungselement in die Verbindungselementaufnahme einführbar ist, in eine Sicherungsstellung überführbar ist, in der das mindestens eine Verbindungselement mittels der Sicherungseinrichtung in der Verbindungselementaufnahme gesichert ist. Es kann zur Erzielung desselben Vorteils auch vorgesehen sein, dass lediglich das vorstehend beschriebene mindestens eine Sicherungsglied von einer derartigen Montagestellung in eine derartige Sicherungsstellung überführbar ist.

Günstig ist es, wenn die Sicherungseinrichtung zum Überführen von der Montagestellung in die Sicherungsstellung zumindest teilweise beweglich am ersten Implantatteil gelagert ist, denn dies erleichtert einem Benutzer die Handhabung der Sicherungseinrichtung.

Vorzugsweise ist die Sicherungseinrichtung zum Überführen von der Montagestellung in die Sicherungsstellung zumindest teilweise in die Verbindungselementaufnahme einführbar. In der Montagestellung kann die Sicherungseinrichtung teilweise oder vollständig außerhalb der Verbindungselementaufnahme angeordnet sein, und diese kann eine Einführöffnung zum Einführen des mindestens einen Verbindungselementes und/oder der Sicherungseinrichtung freigeben. Bei dieser Ausführungsform weist die Sicherungseinrichtung vorzugsweise das vorstehend beschriebene Anlageglied und das vorstehend beschriebene mindestens eine Sicherungsglied auf, so dass das mindestens eine Verbindungselement zum Einführen in die Verbindungselementaufnahme am Anlageglied anliegen kann und durch Überführen der Sicherungseinrichtung von der Montagestellung in die Sicherungsstellung in die Verbindungselementaufnahme eingeführt wird. Deren Einführöffnung kann zum Beispiel mittels des vorstehend beschriebenen mindestens einen Sicherungsgliedes verschlossen werden. Eine derartige Sicherungseinrichtung hat damit gewissermaßen die Funktion einer "Schublade" für das mindestens eine Verbindungselement und ist für einen Benutzer auf besonders einfache Weise handhabbar.

Vorteilhafterweise ist die Sicherungseinrichtung vollständig in die Verbindungselementaufnahme einführbar, wobei sie in der Sicherungsstellung nicht über die äußeren Begrenzungen des ersten Implantatteils hervorsteht. Dies erlaubt es, dem Implantat eine kompakte Bauform zu verleihen. Darüber hinaus ist die Möglichkeit gegeben, eine nachträgliche Manipulation der Sicherungseinrichtung zu vermeiden, wenn sich diese in der Sicherungsstellung befindet. Denn dadurch, dass sie nicht über die äußeren Begrenzungen des Implantatteils hervorsteht, bietet sich einem Benutzer wenig Angriffsfläche, um sie möglicherweise wieder aus der Sicherungsstellung zu entfernen.

Günstig ist es, wenn das erste Implantatteil eine Führung für die Sicherungseinrichtung definiert, denn auf diese Weise kann diese von der Montagestellung in die Sicherungsstellung überführt werden, ohne dass ein Benutzer dabei besonderes Augenmerk auf die Bewegung der Sicherungseinrichtung zu lenken braucht.

Es kann beispielsweise vorgesehen sein, dass die Sicherungseinrichtung durch das erste Implantatteil verschiebbar geführt ist, beispielsweise dann, wenn die Sicherungseinrichtung zum Überführen von der Montagestellung in die Sicherungsstellung zumindest teilweise in die Verbindungselementaufnahme einführbar ist.

Von Vorteil ist es, wenn die Sicherungseinrichtung in der Sicherungsstellung kraft- und/oder formschlüssig in der Verbindungselementaufnahme halterbar ist. Damit lässt sich eine zuverlässige Sicherung des mindestens einen Verbindungselementes in der Verbindungselementaufnahme sicherstellen.

Zur kraft- und/oder formschlüssigen Halterung der Sicherungseinrichtung in der Verbindungselementaufnahme ist es günstig, wenn die Sicherungseinrichtung eine Verriegelungseinrichtung zur Verriegelung der Sicherungseinrichtung in der Sicherungsstellung aufweist. Dadurch ist insbesondere die Möglichkeit gegeben, das Implantat so auszugestalten, dass die Sicherungseinrichtung einmalig von der Montagestellung in die Sicherungsstellung überführbar ist, aber nicht mehr zurück von der Sicherungsstellung in die Montagestellung.

Bei einer konstruktiv einfachen Ausgestaltung des Implantates umfasst die Verriegelungseinrichtung vorteilhafterweise mindestens ein Verriegelungsglied, das in der Sicherungsstellung der Sicherungseinrichtung mit dem ersten Implantatteil in Eingriff steht. Der Eingriff des mindestens einen Verriegelungsgliedes mit dem ersten Implantatteil kann beispielsweise kraft- und/oder formschlüssig ausgebildet sein.

Besonders zuverlässig ist die Sicherungseinrichtung in der Verbindungselementaufnahme mittels der Verriegelungseinrichtung verriegelbar, wenn die Sicherungseinrichtung entlang einer Richtung in die Verbindungselementaufnahme einführbar ist, welche quer und insbesondere senkrecht zur Richtung des Eingriffes des mindestens einen Verriegelungsgliedes mit dem ersten Implantatteil und/oder quer zur Wirkung des Eingriffes ausgerichtet ist. Noch bevorzugter ist die Einführungsrichtung der Sicherungseinrichtung senkrecht zur Richtung des Eingriffes und/oder dessen Wirkung ausgerichtet.

Auf konstruktiv einfache Weise lässt sich die Sicherungseinrichtung fertigen, wenn das mindestens eine Verriegelungsglied als Vorsprung oder als Aufnahme ausgebildet ist, der beziehungsweise die mit einer korrespondierend ausgebildeten Aufnahme beziehungsweise einem korrespondierend ausgebildeten Vorsprung des ersten Implantatteiles zusammenwirkt.

Besonders einfach zu handhaben ist das Implantat, wenn das mindestens eine Verriegelungsglied als Rastelement ausgebildet ist, zum Beispiel als Rastvorsprung oder als Rastaufnahme.

Vorteilhafterweise ist das mindestens eine Verbindungselement in der Verbindungselementaufnahme beweglich gelagert. Dies gibt die Möglichkeit, dass das mindestens eine Verbindungselement relativ zum ersten und/oder zweiten Implantatteil eine Mehrzahl von Positionen einnehmen kann, was bei der Relativausrichtung der beiden Implantatteile von Nutzen sein kann. Das mindestens eine Verbindungselement kann zum Beispiel eine Mehrzahl diskreter Positionen einnehmen. Ergänzend oder alternativ kann es so in der Verbindungselementaufnahme gelagert sein, dass es relativ zu dieser und dem ersten und/oder zweiten Implantatteil stufenlos verstellbar ist. Die beiden Implantatteile lassen sich bei dieser Ausführungsform zum Beispiel beim Einsetzen des Implantates in den Körper eines Patienten den Bedürfnissen des Patienten entsprechend relativ zueinander ausrichten.

Es kann insbesondere vorgesehen sein, dass das mindestens eine Verbindungselement in der Verbindungselementaufnahme verschiebbar gelagert ist.

Um eine zuverlässige Bewegung des mindestens einen Verbindungselementes in der Verbindungselementaufnahme zu ermöglichen, definiert das erste Implantatteil vorzugsweise eine Führung für das in der Verbindungselementaufnahme angeordnete mindestens eine Verbindungselement.

Vorteilhafterweise weist das mindestens eine Verbindungselement mindestens ein Verdrehsicherungsglied auf zur Ausbildung einer Verdrehsicherung des mindestens einen Verbindungselementes relativ zur Verbindungselementaufnahme. Dies gibt beispielsweise die Möglichkeit, eine Ausrichtung des mindestens einen Verbindungselementes relativ zum zweiten Implantatteil aufrechtzuerhalten oder erstmalig zu erzielen. Dies erleichtert einem Benutzer die Verbindung der Implantatteile miteinander. Das mindestens eine Verbindungselement kann zur Verdrehsicherung zum Beispiel formschlüssig zwischen die Verbindungselementaufnahme zumindest teilweise begrenzenden Wandungen des ersten Implantatteiles angeordnet sein.

Das mindestens eine Verdrehsicherungsglied lässt sich beispielsweise als ein Vorsprung des mindestens einen Verbindungselementes ausbilden, welcher an einer Wandung des ersten Implantatteiles, welche die Verbindungselementaufnahme zumindest teilweise begrenzt, anliegt oder mit dieser Wandung in Eingriff steht.

Es ist auch möglich, dass das mindestens eine Verdrehsicherungsglied durch eine Seitenfläche des Verbindungselementes ausgebildet ist, welche beispielsweise an einer die Verbindungselementaufnahme zumindest teilweise begrenzenden Wandung des ersten Implantatteiles anliegt.

Von Vorteil ist es, wenn die Verbindungselementaufnahme mindestens eine Eingriffsöffnung für mindestens ein am zweiten Implantatteil angeordnetes oder anordenbares Verbindungselement aufweist. Die mindestens eine Eingriffsöffnung kann auf diese Weise von dem mindestens einen am zweiten Implantatteil angeordneten oder anordenbares Verbindungselement durchgriffen werden, beispielsweise um es mit dem mindestens einen Verbindungselement des ersten Implantatteiles in Eingriff zu bringen.

Auf konstruktiv einfache Weise lässt sich die mindestens eine Eingriffsöffnung ausbilden, indem die mindestens eine Eingriffsöffnung als Durchbrechung eines Wandbereiches des ersten Implantatteiles ausgebildet ist.

Günstig ist es dabei, wenn der Wandbereich eine Anlagefläche für das zweite Implantat ausbildet. Der Wandbereich kann insbesondere einen Anlageflansch für das zweite Implantatteil ausbilden. Dadurch kann das zweite Implantatteil relativ zum ersten Implantatteil eine definierte Position einnehmen, was nicht nur einem Benutzer die Handhabung des Implantates erleichtert, sondern nach dem Einsetzen des Implantates in den Körper eines Patienten auch dauerhaft zur Sicherung einer definierten Relativausrichtung der beiden Implantatteile geeignet ist.

Vorzugsweise definiert die Durchbrechung ein quer und insbesondere senkrecht zu einer Achse des mindestens einen Verbindungselementes ausgerichtetes Langloch. Das mindestens eine Verbindungselement kann beispielsweise entlang seiner Achse mit einem Verbindungselement des zweiten Implantatteiles verbunden sein. Ist das mindestens eine Verbindungselement in der Verbindungselementaufnahme beweglich angeordnet, zum Bespiel verschieblich in Richtung der Erstreckung des Langloches, können so das erste Implantatteil und das zweite Implantatteil relativ zueinander bewegt und insbesondere verschoben werden.

Wie eingangs erwähnt, betrifft die Erfindung auch eine Kniegelenkendoprothese. Bei einer gattungsgemäßen Kniegelenkendoprothese wird die eingangs genannte Aufgabe erfindungsgemäß dadurch gelöst, dass eine Sicherungseinrichtung zur Sicherung des mindestens einen Verbindungselementes in der Verbindungselementaufnahme vorgesehen ist.

Die erfindungsgemäße Kniegelenkendoprothese weist dann die bereits im Zusammenhang mit der Erläuterung des erfindungsgemäßen Implantates erwähnten Vorteile auf.

Das mindestens eine Implantat der Kniegelenkendoprothese ist günstigerweise als eines der vorstehenden Implantate ausgebildet. Die Kniegelenkendoprothese weist dann die bei der Erläuterung dieser Implantate erwähnten weiteren Vorteile auf.

Das erste Implantatteil des mindestens einen Implantates der Kniegelenkendoprothese kann beispielsweise ein Tibiateil bilden und das zweite Implantatteil einen Tibiaschaft, so dass das mindestens eine Implantat den tibiaseitigen Teil der Kniegelenkendoprothese bilden kann.

Es ist auch möglich, dass das erste Implantatteil ein Femurteil der Kniegelenkendoprothese bildet und das zweite Implantatteil einen Femurschaft, so dass das mindestens eine Implantat den femurseitigen Teil der Kniegelenkendoprothese bilden kann.

Die nachfolgende Beschreibung einer bevorzugten Ausführungsform der Erfindung dient im Zusammenhang mit der Zeichnung der näheren Erläuterung der Erfindung. Es zeigen:

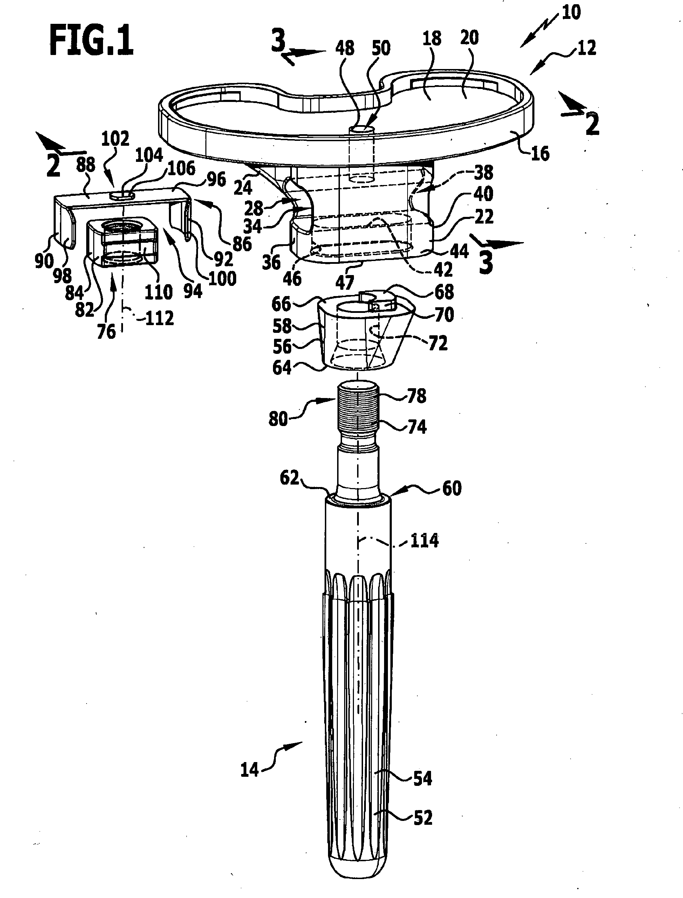

- Figur 1:: eine perspektivische Explosionsdarstellung eines erfindungsgemäßen medizinischen Implantates;
- Figur 2:: eine perspektivische Ansicht des Implantates längs der Linie 2-2 in Figur 1, teilweise in Schnittdarstellung, wobei dessen erstes Implantatteil mit dessen zweitem Implantatteil verbunden ist und
- Figur 3:: eine Schnittansicht längs der Linie 3-3 in Figur 1, wobei dessen erstes Implantatteil mit dessen zweitem Implantatteil verbunden ist.

Eine bevorzugte Ausführungsform eines erfindungsgemäßen Implantates ist in der Zeichnung mit dem Bezugszeichen 10 belegt. Das Implantat 10 bildet den tibiaseitigen Teil einer Kniegelenkendoprothese und umfasst zu diesem Zweck ein erstes Implantatteil 12 sowie ein zweites Implantatteil 14. Das erste Implantatteil 12 bildet ein sogenanntes Tibiateil, welches am proximalen Ende einer Tibia eines Patienten eingesetzt wird. Zur Bildung des Implantates 10 kann es wie untenstehend beschrieben mit dem zweiten Implantatteil 14 zusammenwirken, welches einen sogenannten Tibiaschaft bildet. Das zweite Implantatteil 14 wird distal zum ersten Implantatteil 12 in die Tibia eingesetzt.

Nachfolgend finden über "proximal" und "distal" hinaus auch die weiteren dem Fachmann geläufigen Orts- und Richtungsangaben des Bezugssystems des Patientenkörpers Anwendung.

Das Implantatteil 12 umfasst ein Kopfteil 16 mit einem Auflageabschnitt 18, der eine Auflagefläche 20 für ein Meniskusteil einer Kniegelenkendoprothese bildet, welches an sich bekannt und deswegen in der Zeichnung nicht gezeigt ist. Das Meniskusteil kann in bekannter und in der Zeichnung nicht gezeigter Weise mit einem ebenfalls bekannten und nicht gezeigten Femurteil der Kniegelenkendoprothese zusammenwirken.

Vom Auflageabschnitt 18 steht in distaler Richtung ein Verankerungsabschnitt 22 ab, welcher in eine zuvor präparierte Ausnehmung am proximalen Ende der Tibia eines Patienten eingesetzt werden kann. Der Verankerungsabschnitt 22 ist einstückig mit dem Auflageabschnitt 18 verbunden. Er weist in der Draufsicht ungefähr die Form eines Rechteckes mit abgerundeten Ecken auf, und von ihm stehen in Richtung des Auflageabschnittes 18 seitlich zwei Verstärkungsabschnitte 24 und 26 ab.

Ungefähr auf halber Höhe der Erstreckung des Verankerungsabschnittes 22 in distaler Richtung weist dieser eine senkrecht zur Richtung proximal-distal orientierte und in lateral-medialer Richtung verlaufende Aufnahme auf, welche nachfolgend als Verbindungselementaufnahme 28 bezeichnet wird. Sie wird abschnittsweise in proximaler Richtung von einer Deckenwand 30 und in distaler Richtung von einer Bodenwand 31 begrenzt, die jeweils plan ausgebildet sind und dadurch Ebenen definieren, welche parallel zu der von der Anlagefläche 20 definierten Ebene verlaufen. Posterior und anterior wird die Verbindungselementaufnahme 28 von einer Seitenwand 32 beziehungsweise einer Seitenwand 33 begrenzt, welche die Deckenwand 30 und die Bodenwand 31 miteinander verbinden.

Eine erste Öffnung der Verbindungselementaufnahme 28, nachfolgend als erste Einführöffnung 34 bezeichnet, ist in einem lateralen Wandabschnitt 36 des Verankerungsabschnittes 22 gebildet, und eine zweite Öffnung, nachfolgend als zweite Einführöffnung 38 der Verbindungselementaufnahme 28 bezeichnet, ist in einem medialen Wandabschnitt 40 des Verankerungsabschnittes 22 gebildet.

Von der Bodenwand 31 der Verbindungselementaufnahme 28 erstreckt sich in distaler Richtung eine Durchbrechung 42 die am distalen Ende des Verankerungsabschnittes 22 in einem Wandbereich 44 gebildet ist. Die Durchbrechung 42 weist ferner eine Erstreckung parallel zur Verbindungselementaufnahme 28 von lateral nach medial auf und definiert dadurch ein Langloch 46. Der Wandbereich 44 ist plan ausgebildet und definiert eine Anlagefläche 47 für das zweite Implantatteil 14.

Eine weitere Durchbrechung 48 des Kopfteiles 16 erstreckt sich zwischen der Deckenwand 30 und der Auflagefläche 20 in Form einer zylindrischen Bohrung 50.

Bezüglich einer Ebene von posterior nach anterior, die senkrecht zu der von der Auflagefläche 20 definierten Ebene steht und durch die Mitte der Bohrung 50 verläuft, ist das Kopfteil 16 symmetrisch ausgebildet.

Das mit dem ersten Implantatteil 12 zu verbindende zweite Implantatteil 14 ist zweiteilig ausgebildet und umfasst ein Verankerungsteil 52 in Form eines Schaftes 54 sowie ein auf diesen aufsetzbares Adapterteil 56 in Form eines Kragens 58. Der Schaft 54 ist beim Einsetzen des Implantates 10 in den Körper eines Patienten in eine zuvor in die Tibia präparierte Ausnehmung einzusetzen und bildet das distale Ende des Implantates 10. Er weist nahe seinem oberen Ende eine umlaufende Ringschulter 60 auf, die eine Anlagefläche 62 für eine am Kragen 58 korrespondierend ausgebildete distale Anlagefläche 64 definiert, die ebenfalls ringförmig ausgebildet ist.

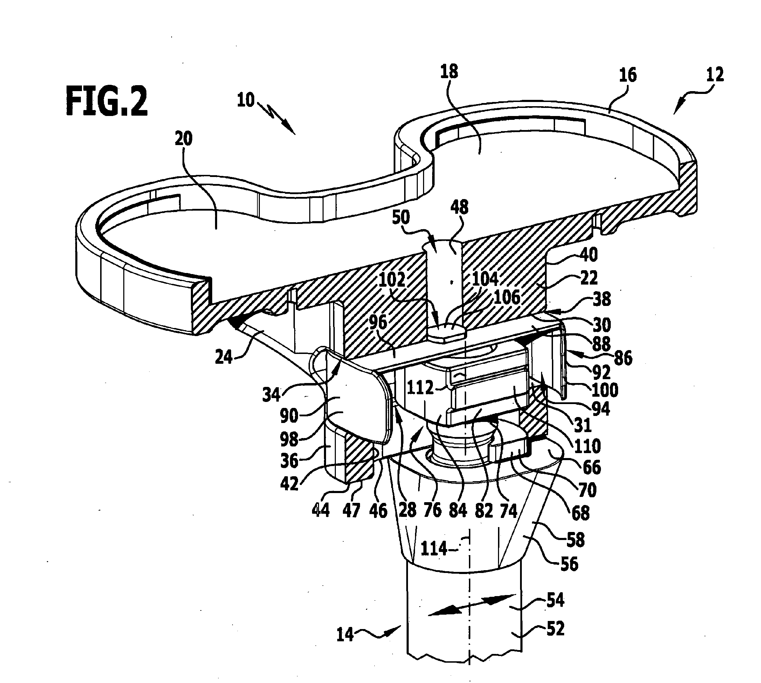

Am proximalen Ende des Kragens 58 ist eine Anlagefläche 66 gebildet, die korrespondierend zu der Anlagefläche 47 des Kopfteiles 16 ausgebildet ist. An der Anlagefläche 66 ist ein Verdrehsicherungsglied 68 angeordnet in Form eines in proximaler Richtung von der Anlagefläche 66 abstehenden Vorsprunges 70, der in die Durchbrechung 42 eintauchen und dabei bündig an deren Wandung anliegen kann. Auf diese Weise besteht zwischen dem Kopfteil 16 und dem Kragen 58 eine Verdrehsicherung, wobei sich der Kragen 58 entlang der Anlagefläche 47 in lateral-medialer Richtung relativ zum Kopfteil 16 bewegen kann, wie dies in Fig. 2 durch einen Doppelpfeil symbolisiert wird, sofern der Kragen 58 nicht wie untenstehend beschrieben am Kopfteil 16 fixiert wird.

Beim Einsetzen des Implantates 10 kann der Schaft 54 mit seinem proximalen Ende eine Durchbrechung 72 des Kragens 58 sowie die Durchbrechung 42 des Kopfteiles 16 durchgreifen, um in die Verbindungselementaufnahme 28 einzugreifen. Auf diese Weise ist damit auch der Schaft 54 relativ zum Kopfteil 16 verschieblich.

Das proximale Ende des Schaftes 54 bildet ein Verbindungselement 74 einer Verbindungseinrichtung 76 des Implantates 10, welche zur Verbindung des Implantatteiles 12 und des Implantatteiles 14 vorgesehen ist. Das Verbindungselement 74 ist vorliegend in Form eines Gewindeabschnittes 78 mit außenliegendem Gewinde 80 ausgestaltet. Der Gewindeabschnitt 78 ist so bemessen, dass er in die Verbindungselementaufnahme 28 zu etwa drei Vierteln eingreifen kann.

Zur Verbindung mit dem zweiten Implantatteil 14 umfasst das erste Implantatteil 12 ein Verbindungselement 82, das ebenso wie das Verbindungselement 74 Bestandteil der Verbindungseinrichtung 76 ist. Es ist in Form einer Mutter 84 ausgebildet, die in die Verbindungselementaufnahme 28 einführbar ist und dort durch Verschrauben mit dem Gewindeabschnitt 78 verbunden werden kann.

Zur Sicherung der Mutter 84 in der Verbindungselementaufnahme 28, das heißt zu deren Halterung und insbesondere beweglichen Halterung darin, umfasst das erfindungsgemäße Implantat 10 und insbesondere dessen erstes Implantatteil 12 eine Sicherungseinrichtung 86, die nachfolgend beschrieben wird.

Die Sicherungseinrichtung 86 umfasst ein längserstrecktes eine Ebene definierendes Element 88, von dessen Enden senkrecht zur Ebene zwei gekrümmte Wände 90 und 92 abstehen. Die Sicherungseinrichtung 86 weist dadurch eine klammerförmige Ausgestaltung auf. Sie ist insgesamt symmetrisch ausgebildet, wobei die Wände 90 und 92 zueinander symmetrisch sind bezüglich einer Symmetrieebene, die senkrecht zu der durch das Element 88 definierten Ebene ist.

Zwischen dem Element 88 sowie den Wänden 90 und 92 ist eine Sicherungsaufnahme 94 für die Mutter 84 definiert, in welche diese auf einfache Weise einführbar ist, da die Sicherungsaufnahme 94 dem Element 88 gegenüberliegend nicht begrenzt ist. In die Sicherungsaufnahme 94 kann die Mutter 84 zum Einführen in die Verbindungselementaufnahme 28 eingebracht werden, wobei das Einführen unter Zuhilfenahme der Sicherungseinrichtung 86 erfolgt. Hierfür kann die Mutter 84 an das Element 88 angelegt werden, so dass dieses ein Anlegeglied 96 der Sicherungseinrichtung 86 definiert. Das Anlegen der Mutter 84 an das Element 88 ist zum Einführen der Mutter 84 in die Verbindungselementaufnahme 28 hilfreich, dafür jedoch nicht unbedingt erforderlich. Es ist ausreichend, wenn die Mutter 84 so in der Sicherungsaufnahme 94 angeordnet wird, dass sowohl die Sicherungseinrichtung 86 als auch die Mutter 84 durch eine der Einführöffnungen 34 oder 38 hindurch in die Verbindungselementaufnahme 28 eingeführt werden kann.

Aufgrund ihrer vorstehend beschriebenen Ausgestaltung bildet die Sicherungseinrichtung 86 gewissermaßen eine "Schublade" für die Mutter 84 zu deren Einführen in die Verbindungselementaufnahme 28. Sie ist dabei von einer Montagestellung, in der die Mutter 84 in die Verbindungselementaufnahme 28 einführbar ist - das heißt zumindest eine der Einführöffnungen 34 oder 38 ist freigegeben - in eine Sicherungsstellung überführbar, in der die Mutter 84 in der Verbindungselementaufnahme 28 gesichert und insbesondere gehalten ist.

Das Einführen der Sicherungseinrichtung 86 mit der Mutter 84 in die Verbindungselementaufnahme 28, das heißt ihr Überführen von der Montagestellung in die Sicherungsstellung ist auf benutzerfreundliche Weise möglich, da die Sicherungseinrichtung 86 und das Kopfteil 16 symmetrisch ausgestaltet sind. Die Sicherungseinrichtung 86 kann dadurch sowohl durch die Einführöffnung 34 als auch durch die Einführöffnung 38 in die Verbindungselementaufnahme 28 eingeführt werden, und zwar jeweils führend mit der Wand 90 oder der Wand 92. Beim Einführen in die Verbindungselementaufnahme 28 wirkt die Deckenwand 30 als Führung für das Element 88. Dies erleichtert einem Benutzer das zielsichere Einführen der Sicherungseinrichtung 86 in die Verbindungselementaufnahme 28.

Ist die Sicherungseinrichtung 86 vollständig in die Verbindungselementaufnahme 28 eingeführt, nimmt sie ihre Sicherungsstellung ein, die Wand 90 fluchtet im Wesentlichen mit dem lateralen Seitenwandabschnitt 36, und die Wand 92 fluchtet im Wesentlichen mit dem medialen Seitenwandabschnitt 40 (Fig.. 2). Wird die Sicherungseinrichtung 86 umgekehrt in die Verbindungselementaufnahme 28 eingeführt, so fluchtet die Wand 90 mit dem medialen Seitenwandabschnitt 40 und die Wand 92 mit dem lateralen Seitenwandabschnitt 36.

Die Wände 90 und 92 sind so bemessen, dass die Einführöffnungen 34 beziehungsweise 38 in der Sicherungsstellung der Sicherungseinrichtung 86 im Wesentlichen vollständig verschlossen werden, so dass zwischen den Wänden 90 und 92 und den Seitenwandabschnitten 36 beziehungsweise 40 nur ein sehr schmaler Fügespalt gebildet ist. Dies hat zweierlei Wirkungen: zum einen ist die Mutter 84 dadurch, dass die Einführöffnungen 34 und 38 verschlossen sind, sicher in der Verbindungselementaufnahme 28 gesichert und kann aus dieser nicht in unbeabsichtigter Weise wegbewegt werden, weswegen die Wände 90 und 92 auch als Sicherungsglieder 98 beziehungsweise 100 der Sicherungseinrichtung 86 bezeichnet werden. Zum anderen ist die Möglichkeit gegeben, dass bei Anwendung des Implantates 10 im Körper eines Patienten außerhalb des Implantates 10 liegendes Material nicht in die Verbindungselementaufnahme 28 gelangen kann. Dabei kann es sich beispielsweise um Knochenzement handeln, der zur Fixierung des Implantatteiles 12 in der Tibia zum Einsatz kommt und gegenüber welchem die Verbindungselementaufnahme 28 durch die Sicherungseinrichtung 86 verschlossen wird.

Die Sicherungseinrichtung 86 kann in der Sicherungsstellung verriegelt werden. Hierfür umfasst sie eine Verriegelungseinrichtung 102, die ein Verriegelungsglied 104 aufweist in Form eines Vorsprunges 106, der vom Element 88 in proximaler Richtung absteht. In der Sicherungsstellung der Sicherungseinrichtung 86 kann der Vorsprung 106 in der Durchbrechung 48 des Kopfteiles 16 verrasten, wobei dessen Eingriffsrichtung in die Durchbrechung 48 senkrecht zur Einführungsrichtung der Sicherungseinrichtung 86 in die Verbindungselementaufnahme 28 ausgerichtet ist. Auf diese Weise ist die Sicherungseinrichtung 86 besonders wirkungsvoll in der Sicherungsstellung verriegelt.

Üblicherweise ist es nicht vorgesehen, die Sicherungseinrichtung 86 wieder von der Sicherungsstellung in die Montagestellung zu überführen. Wenn dies dennoch erwünscht ist, kann ein Benutzer mit einem entsprechenden Stößel oder dergleichen von der Auflagefläche 20 in distaler Richtung in die Durchbrechung 48 eingreifen und den Vorsprung 106 mit einer Kraft in distaler Richtung beaufschlagen, um ihn außer Eingriff mit der Durchbrechung 48 zu bringen. Daraufhin kann die Sicherungseinrichtung 86 wieder aus der Verbindungselementaufnahme 28 heraus geschoben werden.

Bei einer Variante des Implantates 10 kann vorgesehen sein, dass die Sicherungseinrichtung 86 auf andere Weise formschlüssig in der Sicherungsstellung in der Verbindungselementaufnahme 28 gehalten ist, oder dass sie darin kraftschlüssig gehalten ist.

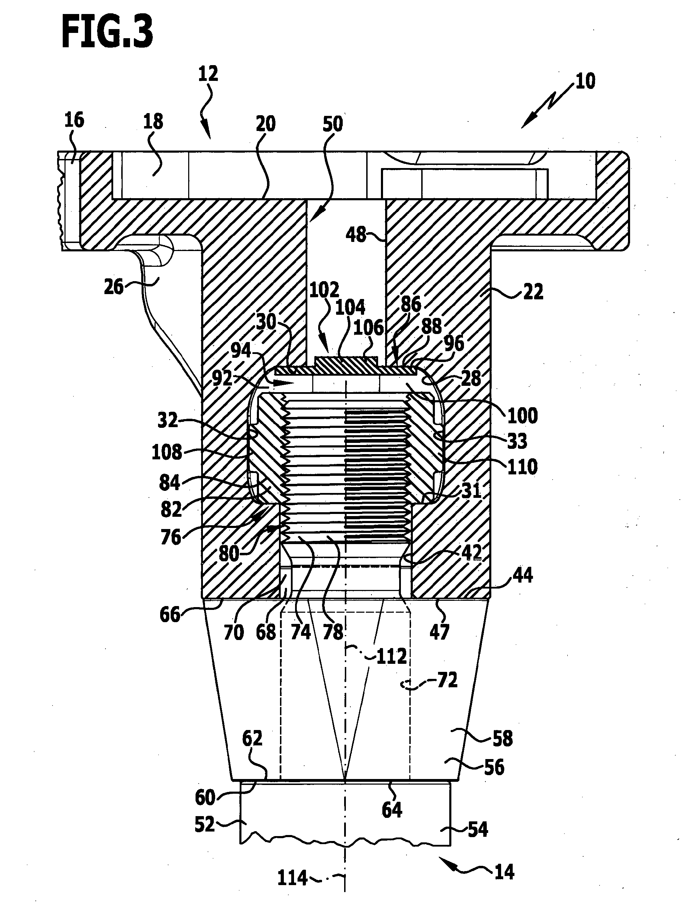

Sowohl in der Sicherungsaufnahme 94 als auch in der Verbindungselementaufnahme 28 ist die Mutter 84 beweglich angeordnet, wobei sie in letzterer insbesondere verschieblich gelagert ist und mittels der Seitenwände 32 und 33 der Verbindungselementaufnahme 28 geführt ist. Zur Führung in der Verbindungselementaufnahme 28 umfasst die Mutter 84 ein Führungsglied in Form einer nach posterior hervorstehenden Rippe 108, die an der Seitenwand 32 anliegen kann, sowie ein Führungsglied in Form einer nach anterior hervorstehenden Rippe 110, die an der Seitenwand 33 anliegen kann (Fig. 3).

Aufgrund dieses Formschlusses zwischen dem Kopfteil 16 und der Mutter 84 in Richtung posterior-anterior wirken die Rippen 108 und 110 gleichzeitig als Verdrehsicherungsglieder, die ein Verdrehen der Mutter 84 in der Verbindungselementaufnahme 28 relativ zum Kopfteil 16 verhindern. Auf diese Weise ist sichergestellt, dass eine Achse 112 der Mutter 84 in der Sicherungsstellung der Sicherungseinrichtung 86 jederzeit senkrecht zu der Anlagefläche 47 für das zweite Implantatteil 14 ausgerichtet ist, so dass sie auf einfache Weise mit einer Achse 114 des Schaftes 54 in Flucht gebracht werden kann. Dies erleichtert einem Benutzer das Ausrichten des Schaftes 54 relativ zur Mutter 84 und damit das Verbinden des ersten Implantatteiles 12 mit dem zweiten Implantatteil 14.

Solange der Gewindeabschnitt 78 nicht fest mit der Mutter 84 verschraubt ist, ist, wie bereits erläutert, der Schaft 54 mit dem Kragen 58 in lateral-medialer Richtung entlang der Anlagefläche 47 verschiebbar. Dies gibt die Möglichkeit, beim Einsetzen des Implantates 10 in den Körper des Patienten die Ausrichtung des zweiten Implantatteils 14 relativ zum ersten Implantatteil 12 entsprechend den Bedürfnissen des Patienten zu wählen (Fig. 2). Wird der Gewindeabschnitt 78 fest mit der Mutter 84 verschraubt, so ist keine Relativbewegung von erstem Implantatteil 12 und zweitem Implantatteil 14 mehr möglich (Fig. 3).

Die erfindungsgemäße Lösung der Aufgabe ermöglicht einem Benutzer somit eine einfache Handhabung des Implantates 10 und gestattet ihm ein zügiges Einsetzen des Implantates 10 in den Körper des Patienten.

Die erfindungsgemäße Lehre kann nicht nur für den tibiaseitigen Teil einer Kniegelenkendoprothese benutzt werden. Es ist auch möglich, dass der femurseitige Teil der Kniegelenkendoprothese, umfassend ein Femurteil sowie einen Femurschaft, entsprechend der erfindungsgemäßen Lehre ausgestattet ist. Darüber hinaus können auch andersartige Implantate, die nicht zur Bildung einer Kniegelenkendoprothese vorgesehen sind, entsprechend der erfindungsgemäßen Lehre ausgestattet sein.

## Patentansprüche

1. Medizinisches Implantat (10) mit einem ersten Implantatteil (12) und einem zweiten Implantatteil (14), zu deren Verbindung eine Verbindungseinrichtung (76) vorgesehen ist, wobei zumindest das erste Implantatteil (12) eine Verbindungselementaufnahme (28) aufweist, in der mindestens ein Verbindungselement (82) der Verbindungseinrichtung (76) anordenbar ist, wobei eine Sicherungseinrichtung (86) zur Sicherung des mindestens einen Verbindungselementes (82) in der Verbindungselementaufnahme (28) vorgesehen ist, wobei die Sicherungseinrichtung (86) mindestens ein Sicherungsglied (98, 100) zur Sicherung des mindestens einen Verbindungselementes (82) in der Verbindungselementaufnahme (28) umfasst, **dadurch gekennzeichnet, dass** die Verbindungselementaufnahme (28) mindestens eine Einführöffnung (34, 38) für das mindestens eine Verbindungselement (82) aufweist, die mit dem mindestens einen Sicherungsglied (98, 100) zumindest teilweise verschlossen ist, wenn die Sicherungseinrichtung (86) eine Sicherungsstellung einnimmt.

2. Implantat nach Anspruch 1, **dadurch gekennzeichnet, dass** die mindestens eine Einführöffnung (34, 38) mittels des mindestens einen Sicherungsgliedes (98, 100) vollständig oder im Wesentlichen vollständig verschließbar ist.

3. Implantat nach Anspruch 1 oder 2, **dadurch gekennzeichnet, dass** die mindestens eine Einführöffnung (34, 38) in einem Wandabschnitt (36, 40) des ersten Implantatteiles (12) gebildet ist und dass das mindestens eine Sicherungsglied (98, 100) zumindest abschnittsweise mit dem Wandabschnitt (36, 40) fluchtet oder im Wesentlichen mit dem Wandabschnitt (36, 40) fluchtet.

4. Implantat nach einem der voranstehenden Ansprüche, **dadurch gekennzeichnet, dass** die Verbindungselementaufnahme (28) zwei Einführöffnungen (34, 38) zum Einführen des mindestens einen Verbindungselementes (82) umfasst und dass die Sicherungseinrichtung (86) zwei Sicherungsglieder (98, 100) aufweist, mit denen jeweils eine Einführöffnung (34, 38) zumindest abschnittsweise verschließbar ist.

5. Implantat nach einem der voranstehenden Ansprüche, **dadurch gekennzeichnet, dass** die Sicherungseinrichtung (86) eine von einer oder mehreren Wandungen (88, 90, 92) zumindest teilweise begrenzte Sicherungsaufnahme (94) für das mindestens eine Verbindungselement (82) ausbildet.

6. Implantat nach Anspruch 5, **dadurch gekennzeichnet, dass** mindestens eine der einen oder mehreren Wandungen (88, 90, 92) der Sicherungseinrichtung (86) die Verbindungselementaufnahme (28) zumindest abschnittsweise begrenzt und/oder dass das mindestens eine Verbindungselement (82) in der Sicherungsaufnahme (94) beweglich angeordnet ist.

7. Implantat nach einem der voranstehenden Ansprüche, **dadurch gekennzeichnet, dass** die Sicherungseinrichtung (86) ausgehend von einer Montagestellung, in der das mindestens eine Verbindungselement (82) in die Verbindungselementaufnahme (28) einführbar ist, in eine Sicherungsstellung überführbar ist, in der das mindestens eine Verbindungselement (82) mittels der Sicherungseinrichtung (86) in der Verbindungselementaufnahme (28) gesichert ist.

8. Implantat nach Anspruch 7, **dadurch gekennzeichnet, dass** die Sicherungseinrichtung (86) zum Überführen von der Montagestellung in die Sicherungsstellung zumindest teilweise in die Verbindungselementaufnahme (28) einführbar ist.

9. Implantat nach Anspruch 8, **dadurch gekennzeichnet, dass** die Sicherungseinrichtung (86) vollständig in die Verbindungselementaufnahme (28) einführbar ist, wobei sie nicht über die äußeren Begrenzungen des ersten Implantatteiles (12) hervorsteht.

10. Implantat nach einem der Ansprüche 7 bis 9, **dadurch gekennzeichnet, dass** die Sicherungseinrichtung (86) eine Verriegelungseinrichtung (102) zur Verriegelung der Sicherungseinrichtung (86) in der Sicherungsstellung aufweist.

11. Implantat nach einem der voranstehenden Ansprüche, **dadurch gekennzeichnet, dass** das mindestens eine Verbindungselement (82) in der Verbindungselementaufnahme (28) beweglich gelagert ist.

12. Implantat nach einem der voranstehenden Ansprüche, **dadurch gekennzeichnet, dass** das mindestens eine Verbindungselement (82) mindestens ein Verdrehsicherungsglied (108, 110) aufweist zur Ausbildung einer Verdrehsicherung des mindestens einen Verbindungselementes (82) relativ zur Verbindungselementaufnahme (28).

13. Implantat nach einem der voranstehenden Ansprüche, **dadurch gekennzeichnet, dass** die Verbindungselementaufnahme (28) mindestens eine Eingriffsöffnung (42) für mindestens ein am zweiten Implantatteil (14) angeordnetes oder anordenbares Verbindungselement (74) aufweist, insbesondere dass die mindestens eine Eingriffsöffnung (42) als Durchbrechung (42) eines Wandbereiches (44) des ersten Implantatteiles (12) ausgebildet ist.

14. Implantat nach Anspruch 13, **dadurch gekennzeichnet, dass** der Wandbereich (44) eine Anlagefläche (47) für das zweite Implantatteil (14) ausbildet.

15. Kniegelenkendoprothese, umfassend mindestens ein Implantat (10) nach einem der voranstehenden Ansprüche.

## Claims

1. A medical implant (10) comprising a first implant part (12) and a second implant part (14), for the connection of which there is provided a connecting device (76), wherein at least the first implant part (12) comprises a connecting element receptacle (28), in which at least one connecting element (82) of the connecting device (76) is disposable, wherein a securing device (86) is provided for securing the at least one connecting element (82) in the connecting element receptacle (28), wherein the securing device (86) comprises at least one securing member (98, 100) for securing the at least one connecting element (82) in the connecting element receptacle (28), **characterized in that** the connecting element receptacle (28) has at least one insertion opening (34, 38) for the at least one connecting element (82), wherein said insertion opening is at least partially closeable by the at least one securing member (98, 100) when the securing device (86) takes a secured position.

2. An implant in accordance with claim 1, **characterized in that** the at least one insertion opening (34, 38) is completely or substantially completely closeable by means of the at least one securing member (98, 100).

3. An implant in accordance with claim 1 or 2, **characterized in that** the at least one insertion opening (34, 38) is formed in a wall section (36, 40) of the first implant part (12), and **in that** at least sections of the at least one securing member (98, 100) are flush with the wall section (36, 40) or are substantially flush with the wall section (36, 40).

4. An implant in accordance with any one of the preceding claims, **characterized in that** the connecting element receptacle (28) comprises two insertion openings (34, 38) for inserting the at least one connecting element (82), and **in that** the securing device (86) has two securing members (98, 100) with which a respective insertion opening (34, 38) is closeable, at least in sections thereof.

5. An implant in accordance with any one of the preceding claims, **characterized in that** the securing device (86) forms a securing receptacle (94) for the at least one connecting element (82) which is at least partly bounded by one or more walls (88, 90, 92).

6. An implant in accordance with claim 5, **characterized in that** at least one of the one or more walls (88, 90, 92) of the securing device (86) bounds the connecting element receptacle (28), at least in sections thereof and/or **in that** the at least one connecting element (82) is arranged moveably in the securing receptacle (94).

7. An implant in accordance with any one of the preceding claims, **characterized in that** the securing device (86) is transferable from an assembly position, in which the at least one connecting element (82) is adapted to be inserted into the connecting element receptacle (28), into a secured position in which the at least one connecting element (82) is secured in the connecting element receptacle (28) by means of the securing device (86).

8. An implant in accordance with claim 7, **characterized in that** the securing device (86) is adapted to be inserted into the connecting element receptacle (28) to at least a partial extent for the transfer from the assembly position into the secured position.

9. An implant in accordance with claim 8, **characterized in that** the securing device (86) is adapted to be inserted entirely into the connecting element receptacle (28), wherein it does not protrude beyond the outer boundaries of the first implant part (12).

10. An implant in accordance with any one of the claims 7 to 9, **characterized in that** the securing device (86) has a locking device (102) for locking the securing device (86) in the secured position.

11. An implant in accordance with any one of the preceding claims, **characterized in that** the at least one connecting element (82) is mounted in the connecting element receptacle (28) in moveable manner.

12. An implant in accordance with any one of the preceding claims, **characterized in that** the at least one connecting element (82) comprises at least one rotation-preventing member (108, 110) for preventing the at least one connecting element (82) from rotating relative to the connecting element receptacle (28).

13. An implant in accordance with any one of the preceding claims, **characterized in that** the connecting element receptacle (28) comprises at least one engagement opening (42) for at least one connecting element (74) which is disposed on or which is disposable on the second implant part (14) in particular, that the at least one engagement opening (42) is formed as a through hole (42) in a wall region (44) of the first implant part (12).

14. An implant in accordance with claim 13, **characterized in that** the wall region (44) forms an abutment surface (47) for the second implant part (14).

15. A knee joint endoprosthesis comprising at least one implant (10) in accordance with any one of the preceding claims.

## Revendications

1. Implant médical (10) comprenant une première partie d'implant (12) et une deuxième partie d'implant (14), pour la liaison réciproque desquelles il est prévu un dispositif de liaison (76), au moins la première partie d'implant (12) présentant un logement de réception d'élément de liaison (28) dans lequel peut être placé au moins un élément de liaison (82) du dispositif de liaison (76), un dispositif de sécurisation (86) étant prévu pour sécuriser ledit au moins un élément de liaison (82) dans le logement de réception d'élément de liaison (28), et le dispositif de sécurisation (86) comprenant au moins un organe de sécurisation (98, 100) pour sécuriser ledit au moins un élément de liaison (82) dans le logement de réception d'élément de liaison (28), **caractérisé en ce que** le logement de réception d'élément de liaison (28) présente au moins une ouverture d'introduction (34, 38) pour ledit au moins un élément de liaison (82), qui est fermée au moins partiellement avec ledit au moins un organe de sécurisation (98, 100), lorsque le dispositif de sécurisation (86) prend une position de sécurisation.

2. Implant selon la revendication 1, **caractérisé en ce que** ladite au moins une ouverture d'introduction (34, 38) peut être fermée entièrement ou sensiblement de manière entière au moyen dudit au moins un organe de sécurisation (98, 100).

3. Implant selon la revendication 1 ou la revendication 2, **caractérisé en ce que** ladite au moins une ouverture d'introduction (34, 38) est formée dans un tronçon de paroi (36, 40) de la première partie d'implant (12), et **en ce que** ledit au moins un organe de sécurisation (98, 100) arrive au ras, au moins par secteurs, du tronçon de paroi (36, 40), ou arrive sensiblement au ras du tronçon de paroi (36, 40).

4. Implant selon l'une des revendications précédentes, **caractérisé en ce que** le logement de réception d'élément de liaison (28) présente deux ouvertures d'introduction (34, 38) pour l'introduction dudit au moins un élément de liaison (82), et **en ce que** le dispositif de sécurisation (86) présente deux organes de sécurisation (98, 100) permettant de fermer respectivement, au moins en partie, une ouverture d'introduction (34, 38).

5. Implant selon l'une des revendications précédentes, **caractérisé en ce que** le dispositif de sécurisation (86) forme un logement de réception de sécurisation (94) destiné au dit au moins un élément de liaison (82), et délimité, au moins en partie, par une ou plusieurs parois (88, 90, 92).

6. Implant selon la revendication 5, **caractérisé en ce que** au moins une desdites une ou plusieurs parois (88, 90, 92) du dispositif de sécurisation (86) délimite, au moins par secteurs, le logement de réception d'élément de liaison (28), et/ou **en ce que** ledit au moins un élément de liaison (82) est agencé de manière mobile dans le logement de réception de sécurisation (94).

7. Implant selon l'une des revendications précédentes, **caractérisé en ce que** le dispositif de sécurisation (86) peut être transféré, à partir d'une position de montage dans laquelle ledit au moins un élément de liaison (82) peut être introduit dans le logement de réception d'élément de liaison (28), à une position de sécurisation dans laquelle ledit au moins un élément de liaison (82) est sécurisé dans le logement de réception d'élément de liaison (28), au moyen du dispositif de sécurisation (86).

8. Implant selon la revendication 7, **caractérisé en ce que** le dispositif de sécurisation (86), pour le transfert de la position de montage à la position de sécurisation, peut être introduit au moins partiellement dans le logement de réception d'élément de liaison (28).

9. Implant selon la revendication 8, **caractérisé en ce que** le dispositif de sécurisation (86) peut être introduit entièrement dans le logement de réception d'élément de liaison (28), en ne dépassant pas des limites extérieures de la première partie d'implant (12).

10. Implant selon l'une des revendications 7 à 9, **caractérisé en ce que** le dispositif de sécurisation (86) comporte un dispositif de verrouillage (102) pour le verrouillage du dispositif de sécurisation (86) dans la position de sécurisation.

11. Implant selon l'une des revendications précédentes, **caractérisé en ce que** ledit au moins un élément de liaison (82) est monté mobile dans le logement de réception d'élément de liaison (28).

12. Implant selon l'une des revendications précédentes, **caractérisé en ce que** ledit au moins un élément de liaison (82) comporte au moins un organe d'arrêt en rotation (108, 110), pour réaliser un arrêt de rotation dudit au moins un élément de liaison (82) par rapport au logement de réception d'élément de liaison (28).

13. Implant selon l'une des revendications précédentes, **caractérisé en ce que** le logement de réception d'élément de liaison (28) comporte au moins une ouverture d'engagement (42) pour au moins un élément de liaison (74) agencé ou pouvant être agencé sur la deuxième partie d'implant (14), notamment **en ce que** ladite au moins une ouverture d'engagement (42) est réalisée sous la forme d'un évidement de passage (42) d'une zone de paroi (44) de la première partie d'implant (12).

14. Implant selon la revendication 13, **caractérisé en ce que** la zone de paroi (44) forme une surface d'appui (47) pour la deuxième partie d'implant (14).

15. Endoprothèse de l'articulation du genou, comprenant au moins un implant (10) selon l'une des revendications précédentes.
